Europäisches Patentamt

**European Patent Office**

**Office européen des brevets**

(19)

(11) Publication number: **0 159 753**
A1

## (12) EUROPEAN PATENT APPLICATION

(21) Application number: **85200548.7**

(22) Date of filing: **04.04.85**

(51) Int. Cl.⁴: **A 61 N 1/05**

(30) Priority: **16.04.84 US 600511**
**06.09.84 US 647975**

(43) Date of publication of application: **30.10.85**
**Bulletin 85/44**

(84) Designated Contracting States: **DE FR GB IT NL**

(71) Applicant: **Astrinski, Eliezer A., 2600-1-N Netherlands Avenue, Riverdale New York 10463 (US)**

(72) Inventor: **Astrinski, Eliezer A., 2600-1-N Netherlands Avenue, Riverdale New York 10463 (US)**

(74) Representative: **Roggero, Sergio et al, Ing. Barzanò & Zanardo Milano S.p.A. Via Borgonuovo 10, I-20121 Milano (IT)**

(54) **Cardiac lead.**

(57) A cardiac sensing and pacing lead (10) primarily for transvenous insertion in the heart and connection to a pacemaker or other measuring or stimulating device. The lead includes a triple electrode configuration (14) wherein three electrodes (16, 18, 20) located axially along the lead (10) are connected such that the two outer electrodes (16, 20) are electrically connected together and to a suitable input port, and the middle electrode (18) is connected to the same input port forming a shielded bipolar sensing element which when inserted in the heart permits strong near field sensing and excellent far field rejection of electrical signals. Epicardial and myocardial lead versions of the above endocardial lead are also described.

EP 0 159 753 A1

0159753

# CARDIAC LEAD

## Field of the Invention

This invention relates to a sensing and pacing lead used with pacemakers or other cardiac measuring or stimulating devices. More particularly, the lead includes a shielded bipolar sensing element comprising a triple electrode configuration which permits strong near field sensing while rejecting far field signals and noise.

## Description of the Prior Art

Heart pacemakers utilize leads that are connected to the heart through a vein. An electrode on the tip of the lead is placed in the ventricle for ventricular pacing. There are medical advantages for many patients to have the ventricular pacing synchronized with the P-wave signal emanating from the atrium. Thus, an atrial synchronous pacemaker was developed to permit this atrio-ventricular synchronization. A separate lead placed in the atrium is required. The additional atrial lead senses the P-wave from the atrium while the ventricular lead is used to correspondingly stimulate or pace the ventricle.

The two types of leads used in the atrium have been unipolar and bipolar. The unipolar lead has not proven satisfactory in synchronous pacing since there is much difficulty in determining the difference between the endocardial P-wave and R-wave as measured in the atrium. Thus, the R-wave may be sensed as a P-wave thereby pacing the ventricle ineffectively as the ventricle cannot respond to stimulation during the refractory period which follows immediately after depolarization. Bipolar leads have proved better for discriminating between P and R-waves, but with the standard two electrode configuration being used a substantial R-wave is still detected which may cause problems distinguishing between atrial and ventricular events.

Thereafter, it was determined that a single lead having an electrode at the tip, for ventricular pacing and electrodes a certain distance away from the tip which would lie in the atrium for atrial sensing would be advantageous.

In U.S. Patent No. 4,365,639, issued to Goldreyer, a single lead having an orthogonal electrode configuration is disclosed. Two pairs of opposed electrodes are mounted circumferentially around the catheter and are connected to produce two bipolar signals sensed from the atrium. A high amplitude P-wave signal is sensed by the Goldreyer configuration with minimum QRS complex amplitudes. A major deficiency of the Goldreyer lead is the difficulty in manufacturing the lead. Furthermore, the electrodes have a small area on the catheter on which to be attached thereby making it a fragile apparatus. In addition, the orthogonal configuration is position sensitive to a point where patient movement may decrease the discriminatory advantages of the system.

An SRT lead, disclosed in CPI brochure No. 4-534-481, has been attempted in a modification of the Goldreyer configuration. A pair of electrodes are angularly spaced by 120° instead of the 180° required by Goldreyer. However, the circumferential location of the pair of electrodes may still present manufacturing difficulties since non-standard electrodes are being used.

U.S. Patent No. 3,659,933 issued to Hagfors is directed to implantable electrodes for nerve stimulation. The electrodes are flexible flat pieces of metal that can be wrapped around a nerve trunk. A guarded electrode configuration is disclosed for providing improved current density. A pair of flat electrodes are connected to one lead and a central, wider flat electrode is connected to a second lead. The leads are encapsulated in a substance inert to body fluids and tissue. The Hagfors electrode apparatus is not designed for endocardial or epicardial use. Hagfors is solely directed to stimulation of nerve trunks dissected out and contained within the electrode insulating sheath. This configuration has also been modified for sensing from isolated nerve trunks.

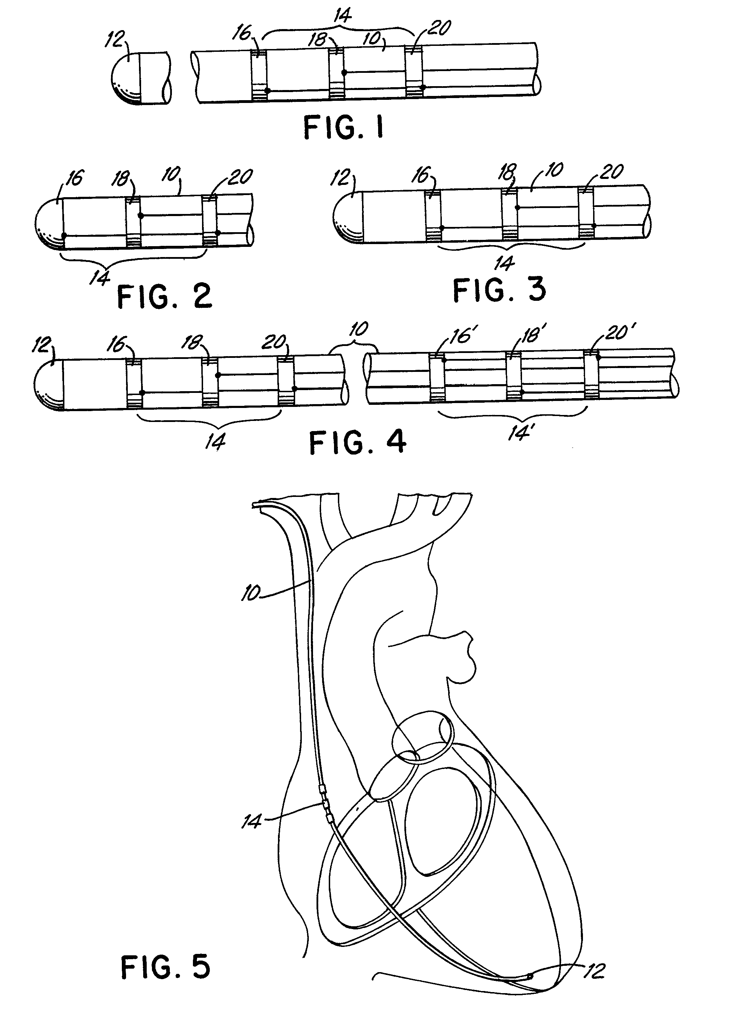

A pill electrode having a quadrupole electrode configuration is disclosed by Arzbaecher in IEEE article 13.4.1, 1982. The quadrupole electrode, as shown in FIGURE 2 of the above article has two positive poles and its two negative poles are combined into one at the center for convenience. The pill electrode is swallowed by the patient and remains in the esophagus immediately posterior to the left atrium. The pill electrode is a detection device used for identification of complex arrhythmia and other monitoring applications. The Arzbaecher electrode is not directed to tranvenous implantation into the heart for atrial and ventricular sensing and pacing.

## Subject of the Invention

Accordingly, it is an object of the subject invention to provide a cardiac lead having a shielded bipolar electrode configuration which provides good near field detection and far field rejection of electrical signals.

It is another object of the subject invention to provide a single endocardial lead that can provide both atrial sensing and ventricular pacing.

In accordance with the invention, there is provided an endocardial lead having a shielded bipolar sensing element comprising first, second and third electrodes located axially along the lead. The lead is inserted in the heart and connected to a pacemaker for atrial or ventricular sensing and pacing. In addition, the lead may be used in connection with measuring devices as a sensing lead.

The first and third electrodes are electrically connected together to form one pole connected to a first terminal. The second or central electrode is connected directly to a second terminal forming the second pole, thereby forming a bipolar element. The first and second terminals may be connected to any measuring or sensing device or to a pacemaker. This triple electrode configuration, unique to endocardial lead systems, provides shielding of the central electrode by the two outer electrodes. Thus, near field cardiac signals are focused and concentrated in the region between the first and third electrodes while far field signals are cancelled.

The triple electrode configuration permits the use of standard ring electrode fabrication technology while providing excellent stability and ease of manufacture.

In addition to the shielded bipolar electrode configuration, a fourth electrode can be provided at the tip of the lead to enable stimulation via the tip electrode and sensing via the shielded bipolar electrode configuration. The endocardial lead according to the invention is particularly adapted for sensing atrial signals while cancelling ventricular artifacts. When the lead is used with an atrial synchronous pacemaker, the lead is introduced transvenously into the heart so that the distal tip electrode is positioned in the apex of the right ventricle and the shielded bipolar sensing element is floating in and adjacent to the wall of the high right atrium. The pacemaker then senses atrial depolarization through the sensing element and can stimulate the ventricle through the tip electrode.

In another embodiment, the single endocardial lead may have two separate shielded bipolar sensing elements, one in the atrium and one in the ventricle for atrial and ventricular sensing together with a ventricular pacing electrode at the distal tip of the lead.

Other embodiments include the lead having the triple electrode configuration and stimulating electrode being used only in the atrium for atrial sensing and pacing. In this case, the sensing element of the subject invention may be utilized on a J lead for atrial sensing or together with a tip electrode for atrial sensing and pacing. Alternatively, the shielded bipolar sensing element and stimulating electrode may be used only in the ventricle for ventricular sensing and pacing.

An alternative to the use of a single electrode for atrial ventricular synchronized pacing is the use of a pair of separate leads as described above, the first for atrial sensing and the second for ventricular pacing. If dual chamber universal pacing is to be implemented (DDD mode) with both atrial and ventricular sensing and pacing, then a J lead with shielded sensing and unipolar pacing elements could be used in the atrium and a separate lead with shielded sensing and unipolar pacing elements could be used in the ventricle.

In addition, the triple electrode configuration may be used for cardiac pacing in either the ventricle or atrium. When used for pacing, there is reduced spread of stimulating current into adjacent tissues compared with unipolar or ordinary bipolar pacing electrodes. Unwanted stimulation of the phrenic nerve or the diaphragm should therefore be reduced.

Furthermore, there is provided an epicardial lead for attachment to the surface of the heart for sensing and or stimulating the heart. The epicardial lead includes a shielded or balanced differential electrode arrangement having three electrode elements mounted on an insulating means. In use, the electrodes are in contact with the heart. In one embodiment of the epicardial lead, the electrodes are arranged in a linear fashion with the outer two electrodes being electrically connected and connected to a first terminal and the third central electrode being connected to a second terminal.

A second embodiment of the epicardial lead includes the electrodes arranged symmetrically in a triangular configuration so that any pair of electrodes can be electrically connected together to the first terminal and the third electrode being connected to the second terminal.

Similarly, a myocardial lead is provided having the three electrodes penetrate the surface of the heart.

## Brief Description of the Drawings

FIGURE 1 is a plan view of the endocardial lead of the invention showing the electrode configuration positioned for atrial sensing and ventricular pacing.

FIGURE 2 is a plan view of the lead of FIGURE 1 showing the electrode configuration positioned at the tip.

FIGURE 3 is a plan view of the lead of FIGURE 1 showing the electrode configuration positioned near the tip.

FIGURE 4 is a plan view of the lead of FIGURE 1 showing the electrode configuration positioned for ventricular sensing and pacing and atrial sensing.

FIGURE 5 is a plan view of the heart showing the placement of the lead of FIGURE 1 of the invention.

FIGURE 6 is an illustration of the electrogram results obtained by the present invention compared to the prior art.

FIGURE 7 is an illustration of the electrogram results obtained by the present invention compared to the prior art when the ventricle is being paced.

FIGURE 8 is a plan view of the heart as in FIGURE 5 showing the electrode configuration of FIGURE 3 positioned for ventricular sensing and pacing.

FIGURE 9 is a view of the heart as in FIGURE 5 showing a J lead version of FIGURE 3.

FIGURE 10 is a plan view of the heart as in FIGURE 5 showing a J version of FIGURE 3 with the electrode configuration positioned for sensing from the high right atrium.

FIGURE 11 is a plan view of the heart as in FIGURE 5 showing a J lead version of FIGURE 2 positioned for atrial sensing.

FIGURE 12 is a plan view of the heart as in FIGURE 5 showing the electrode configuration of FIGURE 4 with the atrial sensing electrodes located in the atrium and the ventricular sensing and pacing electrodes in the ventricle.

FIGURE 13 is a plan view of the epicardial lead of the invention showing the electrodes aligned linearly.

FIGURE 14 is a plan view of another embodiment of the epicardial lead showing the electrodes arranged symmetrically.

FIGURE 15 is a side elevational view of lead of FIGURE 13.

FIGURE 16 is a side elevational view of the myocardial lead of the invention.

## Detailed Description
## of the Preferred Embodiments

Referring now to FIGURE 1, there is shown a preferred embodiment of the lead 10 according to the subject invention. The lead 10 has a stimulating electrode 12 located at the tip of the lead 10. The stimulating electrode 12 is used for ventricular pacing when placed in the ventricle or atrial pacing when placed in the atrium. The stimulating electrode 12 is electrically connected to a pacemaker unit or other stimulating or measuring device (not shown). A three ring bipolar sensing element 14 is located on the lead 10 and comprises electrodes 16, 18 and 20 located axially along the lead 10. The electrodes 16, 18 and 20 are substantially equi-distant from each other. The center to center distance between the electrodes can be from one centimeter to as close as one millimeter. The preferred distance is a 3.5 mm center to center separation. The axial length of each ring electrode can be from 0.1 to 10 mm. The preferred length is 1.5 mm. The rings may be of different lengths but the preferred embodiment has equal rings. The central electrode 18 is electrically connected to a first terminal of the pacemaker unit or other measuring device. The outside electrodes 16 and 20 are electrically connected together and are also electrically connected to a second terminal of the pacemaker unit or measuring device. Hence, the three electrodes 16, 18 and 20, configured in this manner form the shielded bipolar sensing element 14. The outer two electrodes 16 and 20 being connected together provide shielding of the central electrode 18. Near field signals, originating from the section of the heart immediately adjacent to the sensing element 14, are detected while far field signals, originating from distant sections of the heart or elsewhere, are blocked or cancelled.

Furthermore, there may be applications such as where rejection of pacemaker pulses originating close to the sensing element 14 is essential where one of the outside electrodes 16 or 20 is at a greater distance from the central electrode 18 than the distance between the other outside electrode and the central electrode. 18. In these applications, there will also be excellent blocking of far field signals and detection of near field signals by the shielded sensing element 14. In addition, a pair of resistors of substantially equal resistance may be placed in the line connecting electrodes 16 and 20. One of the resistors will be placed between the electrical connection point and electrode 16 and the other will be between the electrical connection point and the electrode 20.

In the embodiment of FIGURE 1, the shielded bipolar sensing element 14 is used for atrial sensing and is located at a position on the lead 10 such that the element 14 lies in the atrium of the patient's heart. FIGURE 5 shows the preferred embodiment after transvenous insertion in the heart wherein the stimulating electrode 12 is located in the right ventricle and the bipolar sensing element 14 is located in the right atrium. Thus, a strong atrial signal corresponding to a surface ECG's P-wave will be detected by sensing element 14. At the same time, ventricular depolarization, myopotentials and other electrical signals originating from distant sections of the heart or body will be rejected thereby permitting accurate interference-free atrial synchronous ventricular pacing.

The electrodes 12, 16, 18 and 20 can be made of materials suited for implantation such as platinum or platinum-iridium, carbon, or an alloy known by the trade name of Elgiloy commercially available from the Elgin
Watch Co.

Sensing element 14 can be located anywhere
along the lead 10 including the distal end.  FIGURE 2
shows the sensing element 14 wherein the outside electrode
16 is located at the distal end of the lead.  The
embodiment of FIGURE 2 may be used for either ventricular
or atrial sensing.  The bipolar element 14 may also be
used for pacing.  The stimulating currents are focused
in the region between electrodes 16 and 20 without
significant spreading experienced with unipolar or the
standard bipolar pacing electrode.

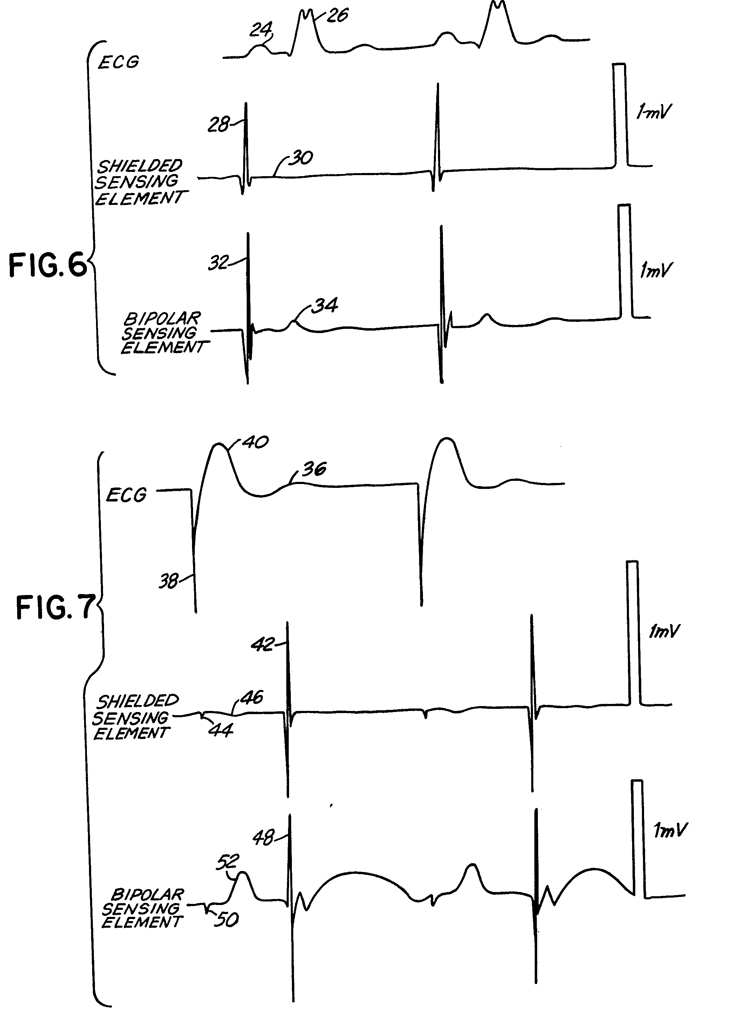

FIGURE 6 is an illustration of the results
obtained when the lead 10 is used for atrial sensing.
The bipolar sensing element 14, by transvenous insertion
is placed in the atrium.  The upper tracing is an
electrocardiogram showing the surface ECG's P-wave 24
and the QRS wave 26.  The central tracing shows the
endocardial P-wave 28 and the endocardial QRS wave 30
as sensed by the sensing element 14.  As can be seen,
the QRS wave is practically negligible, while the P-wave
is a distinct easily detectable atrial signal.  The
lower tracing shows the results using a standard bipolar
electrode placed in the atrium   The P-wave 32 is not as
sharp a signal as with the subject invention and the far
field QRS 34 is significantly larger.  The central
tracing illustrates the improved signal to noise ratio
as compared with the lower tracing.

FIGURE 7 is an illustration of the results
obtained when the lead 10 is inserted in the atrium and
a separate lead is used to pace the ventricle.  The
upper trace is the surface ECG showing the retrograde P-wave 36, ventricular pacer pulse 38 and the QRS 40. The middle trace shows the sharp endocardial P-wave 42 sensed by the sensing element 14, a near zero pacer artifact 44 and the minimal endocardial QRS 46. The sensing element 14 almost completely rejects the ventricular pacer pulse. The lower trace shows the results using a standard bipolar lead showing the P-wave 48, ventricular artifact in the atrium 50 and the endocardial QRS in the atrium 52. The shielded bipolar sensing element therefore provides superior rejection of far field ventricular pacing pulses and R-wave signals originating in the ventricle, while clearly detecting the P-wave in the atrium.

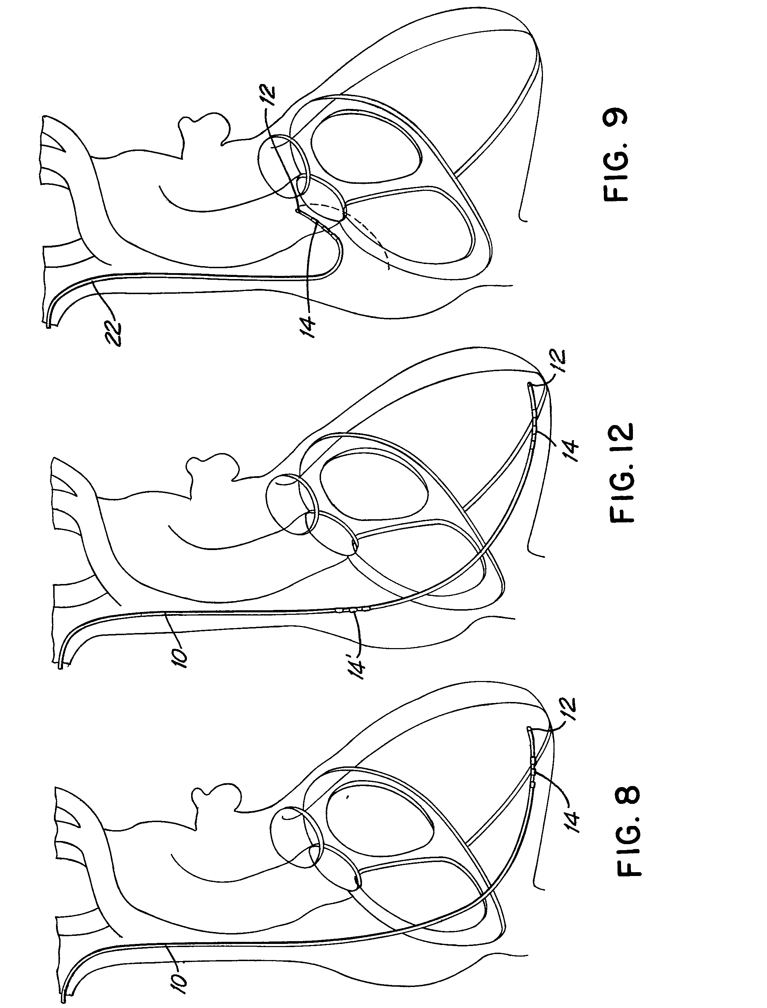

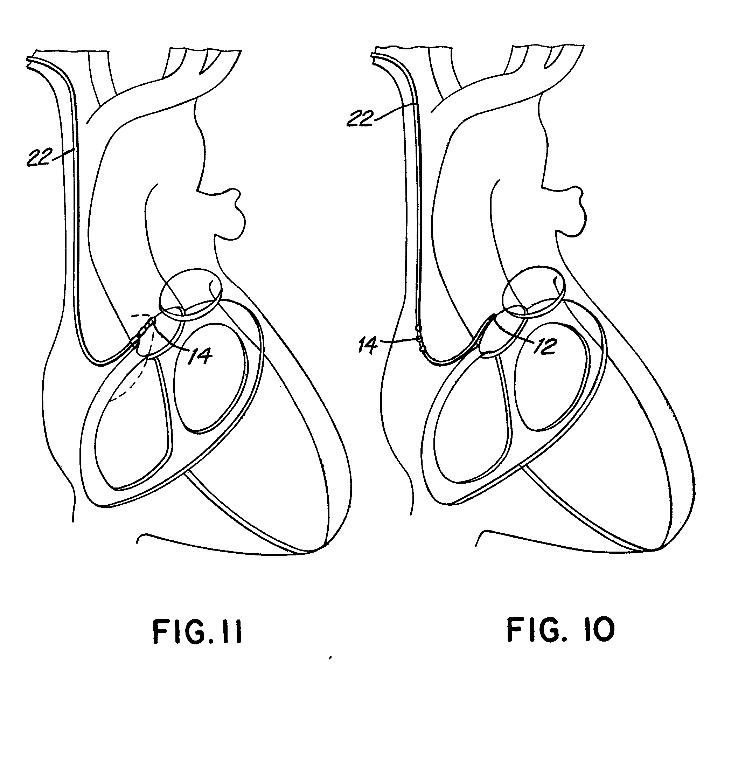

In another embodiment shown in FIGURE 3, the sensing element 14 is placed near the tip 12 which allows for both pacing and sensing in the same chamber. For example, by placing the lead 10 shown in FIGURE 3 in the ventricle, as in FIGURE 8, ventricular pacing and sensing can be performed. The embodiment of the invention as shown in FIGURE 3 will permit sensing of ventricular depolarization immediately after pacing without the need for pacemaker rapid recharge circuits and long blanking periods. The same will occur when the lead 10 is placed in the atrium.

For atrial use, FIGURE 9 shows the positioning of the sensing element 14 and the pacing element 12 when incorporated in a J lead 22 in the right atrial appendage. Further, as shown in FIGURE 10, the J lead 22 may include the stimulating tip 12 located in the right atrial appendage and the sensing element 14 located on the J lead 22 such that the sensing element 14 is floating in the right atrium.

FIGURE 11 shows the positioning of J lead 22, wherein one electrode of the sensing element 14 is located at the tip of the J lead 22 in the right atrial appendage.

The embodiment of FIGURE 4 shows the lead 10 having the sensing element 14 positioned as in FIGURE 3 with an additional sensing element 14' located away from the tip 12 as in FIGURE 1. Thus, with the tip 12 and the sensing element 14 placed in the ventricle and the sensing element 14' placed in the atrium, there is provided a means for atrial sensing, ventricular sensing and ventricular pacing. This arrangement is shown in FIGURE 12.

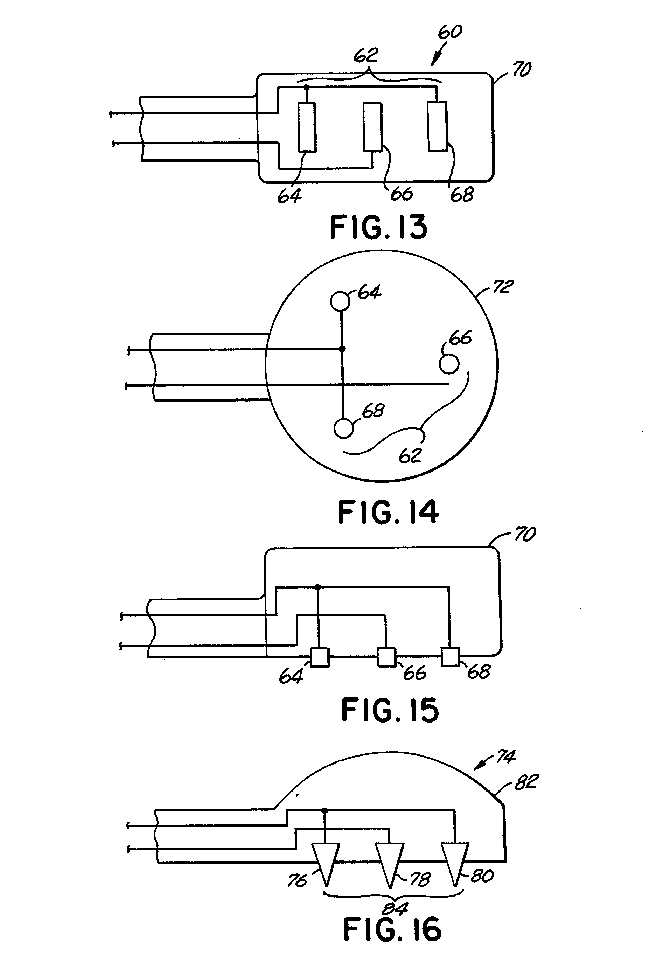

The epicardial lead of the invention is shown in FIGURES 13, 14 and 15. The epicardial lead 60 of FIGURE 13 includes the shielded sensing element 62 with the electrodes 64, 66 and 68 aligned linearly and mounted on an insulating means 70. The electrodes 64 and 68 are electrically connected together and to a first terminal of the cardiac measuring or stimulating device (not shown). The electrode 66 is electrically connected to a second terminal of the cardiac device. The electrodes 64, 66 and 68 of the epicardial lead 60 protrude on end of the insulating means 70 in order to contact the surface of the heart as shown in FIGURE 15.

The electrodes 64, 66 and 68 may be arranged symmetrically as in FIGURE 14 on an epicardial lead having a circular insulating means 72. In FIGURE 14, electrodes 64 and 68 are electrically connected together and connected to a first terminal of the cardiac measuring or stimulating device (not shown) being used and electrode 66 is electrically connected to a second terminal of the cardiac device. However, in this embodiment, any two electrodes may be electrically connected to form the bipolar element 62 and still have the properties of excellent detection of near field signals and cancelling of far field signals.

FIGURE 16 shows a myocardial lead 74 wherein the electrodes 76, 78 and 80 protrude from one end of the insulating means 82 in a sharp point that pierces and grips the heart upon insertion. The shielded sensing element 84 is formed by electrically connecting electrodes 76 and 80 as discussed above.

The invention should not be limited to use with a pacemaker, as it can be used with any measuring or stimulating device. In addition to the uses stated above, the lead according to the invention may be used for recording from and accurate location of electrical pathways or electrically active tissues in the heart such as the Bundle of His, Atrio-Ventricular Node, a Kent Bundle, and a source of ectopic electrical activity. Once located, the invention may be used selectively for stimulating or for ablation of these tissues by passing current through the electrodes to these tissues.

While preferred embodiments of the subject invention have been described and illustrated, it is obvious that various changes and modifications can be made therein without departing from the spirit of the present invention which should be limited only by the scope of the appended claims.

CLAIMS:

1. An endocardial lead for connection between a pacemaker and a heart comprising:

a triple electrode configuration comprising three electrodes located axially along the lead, said three electrodes include two outer electrodes and one inner electrode, said outer electrodes being electrically connected together and also connected to said pacemaker, and said inner electrode being electrically connected to said pacemaker thereby forming a shielded bipolar sensing element which permits strong near field sensing and far field rejection of electrical signals when said lead is inserted in the heart.

2. A lead as in Claim 1 wherein said lead further includes a stimulating electrode located at the tip of said lead, said stimulating electrode being electrically connected to said pacemaker.

3. An endocardial lead for connection between a measuring device and a heart comprising:

a triple electrode configuration comprising three electrodes located axially along the lead, said three electrodes include two outer electrodes and one inner electrode, said outer electrodes being electrically connected together and also connected to a first terminal of said measuring device, and said inner electrode being electrically connected to a second terminal of said measuring device thereby forming a shielded bipolar sensing element which permits strong near field sensing and far field rejection of electrical signals when said lead is inserted in the heart.

4.    A lead as in Claim 3 wherein said lead further includes a stimulating electrode located at the tip of said lead.

5.    A lead as in Claims 2 or 4 wherein the distance between said stimulating electrode and said triple electrode configuration being substantially equal to the distance between the right ventricle and right atrium of said heart.

6.    A lead as in Claims 2 or 4 wherein the distance between said stimulating electrode and said triple electrode configuration is such that when said lead is inserted in said heart, both the stimulating electrode and the triple electrode configuration will be located in the same chamber of said heart.

7.    A lead as in Claims 1 or 3 wherein one of the said outer electrodes is located at the distal end of said lead.

8.    An endocardial lead for connection between a pacemaker and a heart comprising:
    a plurality of triple electrode configurations, each of said triple electrode configurations including three electrodes located axially along said lead;
    each of said triple electrode configurations including two outer electrodes and one inner electrode, said outer electrodes being electrically connected together and to said pacemaker, and said inner electrode being electrically connected to said pacemaker thereby forming a plurality of shielded bipolar sensing elements which permit strong near field sensing and far field rejection of electrical signals when said lead is inserted in said heart; and

a stimulating electrode located at the tip of said lead.

9. An endocardial lead for connection between a measuring device and a heart comprising:

a plurality of triple electrode configurations, each of said triple electrode configurations including three electrodes located axially along said lead;

each of said triple electrode configurations including two outer electrodes and one inner electrode, said outer electrodes being electrically connected together and to a first terminal of said measuring device, and said inner electrode being electrically connected to a second terminal of said measuring device thereby forming a plurality of shielded bipolar sensing elements which permit strong near field sensing and far field rejection of electrical signals when said lead is inserted in said heart; and

a stimulating electrode located at the tip of said lead.

10. A lead as in Claims 8 or 9 wherein a first triple electrode configuration is located at a distance from the stimulating tip equal to the distance between the right ventricle and the right atrium of said heart and a second triple electrode configuration is located at a distance from the stimulating electrode such that when the tip of said lead is inserted in the right ventricle of the said heart, the second triple electrode configuration will be located in the right ventricle and the first triple electrode configuration will be located in the right atrium.

11. A lead as in Claims 1, 3, 8 or 9 wherein the three electrodes of the triple electrode configuration are spaced substantially equi-distant from each other.

12. A lead as in Claims 1, 3, 8 or 9 wherein the electrodes are composed of platinum.

13. A lead as in Claims 1, 3, 8 or 9 wherein the electrodes are composed of carbon.

14. A lead as in Claims 1, 3, 8 or 9 wherein the electrodes are composed of Platinum-Iridium.

15. A lead as in Claims 1, 3, 8 or 9 wherein the electrodes are composed of Elgiloy.

16. A lead as in Claims 1 or 3 wherein the triple electrode configuation is located on a J-lead.

17. A lead as in Claim 6 wherein the stimulating electrode and the triple electrode configuration are located on a J-lead.

18. A lead as in Claim 17 wherein the stimulating electrode is located at the tip of the J-lead and the triple electrode configuration is located on the J-lead at a distance from the tip such that the triple electrode configuration is floating in the right atrium.

19. A lead as in claim 7 wherein the triple electrode configuration is located on a J-lead.

20. A lead as in claim 2 wherein the stimulating electrode and the triple electrode configuration is located on a J-lead.

21. An epicardial lead for connection between a heart and an electrical device comprising:

an insulating means and a triple electrode configuration comprising three electrodes mounted axially on said insulating means, said electrodes extending through one end of said insulating means for contacting the surface of the heart, said three electrodes include two outer electrodes and one inner electrode, said outer electrodes being electrically connected together and also connected to a first terminal of said electrical device and said inner electrode being electrically connected to a second terminal of said electrical device thereby forming a shielded bipolar sensing element which permits strong near field sensing and far field rejection of electrical signals.

22. An epicardial lead for connection between heart and an electrical device comprising:

an insulating means and a triple electrode configuration comprising three electrodes mounted symmetrically on said insulating means, said electrodes extending through one end of said insulating means for contacting the surface of the heart, two of said electrodes being electrically connected together and also connected to a first terminal of said electrical device and said third electrode being electrically connected to a second terminal of said electrical device thereby forming a shielded bipolar sensing element which permits strong near field sensing and far field rejection of electrical signals.

23. A myocardial lead for connection between a heart and electrical device comprising:

an insulating means and a triple electrode configuration comprising three electrodes mounted on said insulating means, said electrodes extending through said insulating means in a point for embedding in the heart wall, two of said electrodes being electrically connected together and also connected to a first terminal of said electrical device and said third electrode being electrically connected to a second terminal of said electrical device thereby forming a shielded bipolar sensing element which permits strong near field sensing and far field rejection of electrical signals.

0159753

FIG. 1

FIG. 2

FIG. 3

FIG. 4

FIG. 5

0159753

215

FIG.6

ECG

24  26

SHIELDED
SENSING
ELEMENT

28  30  1mV

BIPOLAR
SENSING
ELEMENT

32  34  1mV

FIG.7

ECG

40  36  38

SHIELDED
SENSING
ELEMENT

42  46  44  1mV

BIPOLAR
SENSING
ELEMENT

48  52  50  1mV

0159753

415

FIG. 11

FIG. 10

**FIG. 13**

**FIG. 14**

**FIG. 15**

**FIG. 16**

# EUROPEAN SEARCH REPORT

## DOCUMENTS CONSIDERED TO BE RELEVANT

EP 85200548.7

| Category | Citation of document with indication, where appropriate, of relevant passages | Relevant to claim | CLASSIFICATION OF THE APPLICATION (Int. Cl 4) |
|---|---|---|---|
| X | WO - A1 - 80/2 801 (REENSTIERNA) | 1-5 | A 61 N 1/05 |
| A | * Abstract; page 2, lines 7-28; page 3, lines 1-6; fig. 1-3 * | 8,9, 12,14, 21 | |
| | -- | | |
| X | EP - A1 - 0 024 913 (SOWTON) | 22,23 | |
| | * Abstract; page 2, lines 18-30; fig. 1,2 * | | |
| | -- | | |
| Y | US - A - 3 825 015 (BERKOVITS) | 1,3,8-10,21,23 | |
| A | * Column 2, line 47 - column 3, line 53; fig. 1 * | 2,4-7, 12,14 | |
| | -- | | |
| Y | DD - A - 18 488 (WEISSENBORN) | 1,3,8-10,21,23 | |
| | * Page 3, lines 26-29, 45-59; fig. * | | |
| | -- | | |
| A | EP - A1 - 0 009 732 (PRECIMED) | 22 | |
| | * Page 3, lines 26,27; page 4, lines 7,8; fig. 1,2 * | | |
| | -- | | |
| A | WO - A1 - 81/3 428 (PLESS) | 1,3,8, 9,11, 21,23 | |
| | * Abstract; page 10, lines 21-28; fig. 1,2 * | | |
| | -- | | |

TECHNICAL FIELDS SEARCHED (Int. Cl 4)

A 61 N
A 61 B
A 61 M

The present search report has been drawn up for all claims

| Place of search | Date of completion of the search | Examiner |
|---|---|---|
| VIENNA | 10-07-1985 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

## EUROPEAN SEARCH REPORT

| | DOCUMENTS CONSIDERED TO BE RELEVANT | | EP 85200548.7 |
|---|---|---|---|
| Category | Citation of document with indication, where appropriate, of relevant passages | Relevant to claim | CLASSIFICATION OF THE APPLICATION (Int. Cl 4) |
| Y,D | US - A - 4 365 639 (GOLDREYER) | 1,3,9, 11,21, 23 | |
| A | * Abstract; fig. 10-15 * | 2,4-8, 10,16- 20,22 | |
| | -- | | |
| Y | DE - B2 - 2 140 994 (SIEMENS) * Fig. * | 1,3,9, 11,21, 23 | |
| | -- | | |
| A | DE - A1 - 3 327 561 (VEB TRANS-FORMATOREN- UND RÖNTGENWERK "HERMANN MATERN") * Page 5, lines 7-14; fig. * | 1,3,8, 9,21 | |
| | -- | | |
| Y | US - A - 4 379 462 (BORKAN) * Column 2, lines 52-61; fig. 1,3 * | 1,3,8, 9,21 | TECHNICAL FIELDS SEARCHED (Int. Cl 4) |
| | -- | | |
| Y | US - A - 4 172 451 (KLINE) * Column 2, lines 51-58; fig. 2 * | 1,3,8, 9,21 | |
| | ---- | | |

The present search report has been drawn up for all claims

| Place of search VIENNA | Date of completion of the search 10-07-1985 | Examiner NEGWER |
|---|---|---|